# EUROPEAN PATENT APPLICATION

(11) **EP 0 867 452 A1**
(43) Date of publication of application: **30.09.1998**
(21) Application number: 97944127.6
(22) Date of filing: 15.10.1997
(51) Int. Cl.: C08B 37/08, A61K 31/725

(54) **CERVICAL CANAL MATURING AGENT**

(30) Priority: 15.10.1996 JP 272554/96
(71) Applicant: TORAY INDUSTRIES, INC., Tokyo 103 (JP)
(72) Inventor: TERAO, Toshihiko, Shizuoka 431-31 (JP); KANAYAMA, Naohiro Hamamatsuidaihandayamashukusha, Hamamatsu-shi Shizuoka 431-31 (JP); IDA, Nobutaka, Kanagawa 248 (JP)
(74) Representative: Kador & Partner
(86) International application number: JP9703722
(87) International publication number: WO9816559

(57) **Abstract**

A cervical ripening agent contains as an active ingredient hyaluronic acid and/or a hyaluronic acid derivative. The present invention discloses the cervical ripening action of hyaluronic acid and/or a hyaluronic acid derivative, and exhibits effectiveness as a medicine and application means for not only parturition and abortion treatment but also treatment related to artificial fertilization.

## Description

### Technical Field

The present invention relates to a uterine cervical ripening agent containing as an active ingredient hyaluronic acid and/or a hyaluronic acid derivative having the action to facilitate ripening of the uterine cervix and the action to ensure the parturient canal to assist expulsion of a fetus in parturition.

### Background Art

A normal birth process is mainly controlled by two physiological processes which proceed with appropriate timing. Namely, the two processes include contraction of the uterine muscle for extracting a fetus, and ripening and dilation of the uterine cervix serving as an expulsion route of a fetus. Insufficient proceeding of these processes or improper linkage therebetween induces difficult parturition. As means for coping with such a state, chemical, medical manipulation or physical expulsive means such as an oxytocic such as prostaglandin or the like, Kristeller maneuver, suction and forceps surgery or the like has conventionally been used. When these means exhibit no effect, Caesarean section is conducted. At present, Caesarean section is safely conducted, but the mortality of mothers and children is by no means low. Particularly, the mortality of mothers is significantly high as compared with parturition through the natural parturient canal. Therefore, parturition through the natural parturient canal, i.e., transvaginal parturition, is preferred as long as there is an appropriate method.

Typical physiological factors related to uterine contraction include oxytocin and prostaglandin, which have already been clinically used as therapeutic agents for oxytocic failure in an intrapartum period. Also, ergometrine maleate, methylergometrine maleate, sparteine sulfate, etc. are also used as oxytocics at medical sites.

On the other hand, even if oxytocic force is normally controlled, insufficient ripening of the uterine cervix makes it impossible to ensure the parturient canal and thus results in a situation in which the lives of a mother and a child are jeopardized due to prolongation of parturition or hysterorrhexis. A typical example of conventional means for dilating the uterine cervix is a physical extension method using a plant such as Laminaria Japonica or a synthetic resin or polymer absorber such as Lamicel (trade name), Dailapan (trade name) or the like for the cervical region. These are cervical dilators which are cylindrical (a rod shape) and inserted into the uterine cervix to dilate the cervix by the force of expansion of the carrier volume due to water absorption. Various modified techniques of the physical extension method are disclosed (Shibano et al.: Japanese Unexamined Patent Publication No. 63-267370, Maruoka et al.: Japanese Unexamined Patent Publication No. 2-92367, Yamada et al.: Japanese Unexamined Patent Publication No. 2-220667). A chemical method uses a benzoate or propionate of estriol as an estrogen formulation, or prasterone sodium sulfate (trade name; Mylis) as a formulation acting on the uterine cervix. The action of ripening the uterine cervix has recently been found in various peptides, e.g., interleukin 8, interleukin 1β, platelet factor 4, and the like, and the potential for medicines is expected. The most significant biochemical changes in ripening of the uterine cervix are decreases in collagen and glucosaminoglucan and an increase in hyaluronic acid inversely proportional thereto (von Malliot, K. et al, Am. J. Obstet. Gynecol., 135,503, 1979, Golichowski, in: Dilatation of Uterine Cervix (eds) Naftolin, F. and Stubblefield, P. G., p. 99, Raven Press, New York, 1980).

Hyaluronic acid is a natural polysaccharide composed of alternate residues of D-glucuronic acid and N-acetyl-D-glucuronic acid (Laurent, T. C. and Fraser, R. E., in: Functions of the Proteoglicans, eds, D. Everd and J. Whelan, pp. 9-14, John Wiley). This is a linear polymer having a wide range of molecular weights depending upon the origin of preparation, the production method and the method of measuring the molecular weights. Hyaluronic acid is naturally present in the gel around a cell, as a main component of the basic substance of the connective tissue of a vertebrate, in the articular synovial fluid, in the vitreous humors of the eyes, in the umbilical cord tissues, and in the cock's crista galli. Hyaluronic acid is synthesized by the fibroblasts of the human cervical tissue (Tanaka, K. et al., FEBS Letters, 347, 95, 1994). It has been known that synthesis of hyaluronic acid is accelerated by various stimulations with hormones (Tanaka, K. et al., FEBS Letters, 347, 95, 1994), prostaglandin (Rath, W. et al., Prostaglandins, 45, 375, 1993), IL-1 (Ito, A. et al., Biochim. Biophys. Acta., 1158, 91, 1993), lipopolysaccharides (Laurent, T. C. and Fraser, R. E., in: Functions of Proteoglicans, (eds) Everd, D. & Whelan, J., p. 9 , John Wiley, Chichester, England, 1986), and the like. Although the most famous biological function of hyaluronic acid is the water holding property in tissues (Comper, W. D. and Laurent, T. C., Physiol. Rev., 58, 255, 1978), the facts that hyaluronic acid is also related to cell proliferation (Tomida M. et al., J. Cell Physiol., 86, 121, 1975), cell migration (Toole, B. P., Dev. Biol., 29, 321, 1972), irruption into tissues (Kunudson, W. et al., Proc. Natl. Acad. Sci., USA, 81, 6767, 1984), transformation (Hopwood, J. J. and Dorfman, A., J. Biol. Chem., 252, 4777, 1977), mitoses (Brecht et al., Biochem. J., 239, 445, 1986), vascularization (West, D. C. et al., Science, 228, 1324, 1985), and wound healing (Masst, B. A. et al., Plastic and Reconstructive Surgery, 89, 503, 1992) are increasingly accumulated.

The contents of reports on the relation between cervical ripening and hyaluronic acid are the following. Hyaluronic acid is present in the cervical regions of the human pregnant uterus and non-pregnant uterus. Also, the local concentration of hyaluronic acid in the uterine cervix increases 2 to 12 times with ripening of the cervical region of the uterus (von Maillot, K. et al., Am. J. Obstet. Gynecol., 135, 503, 1979, Osmers, R. et al., Obstet. Gynecol., 81, 88, 1993). Besides an increase in the concentration of hyaluronic acid in the cervical region in the natural ripening process accompanying proceeding of pregnancy, the content of hyaluronic acid in the uterine cervix is increased by administering a substance known to induce ripening, for example, prostaglandin E2 (Rath, W. et al., Prostaglandins, 45, 375, 1993), antiprogesterone (Cabrol, D. et al., Prostaglandins, 42, 71, 1991) or relaxin (Downing, S. J. and Sherwood, O. D., Endocrinol., 118, 471, 1986). Namely, hyaluronic acid is known to be a good marker for ripening of the uterine cervix. On the other hand, in regard to the relation between hyaluronidase which is a degradation enzyme of hyaluronic acid and the uterine cervix, it has been reported that the human or animal uterine cervix can be ripened by administering hyaluronidase which is an enzyme for decomposing hyaluronic acid (Gupta, T. et al., J. Indian Med. Association, 92, 47, 1994, Li, W. J. et al., Chinese Med. J., 107, 552, 1994, Li, Z. et al., Chinese J. Obstet. Gynecol., 28, 292, 1993). It has been recognized that an increase in the concentration of hyaluronic acid with ripening of the uterine cervix is a phenomenon related directly to softening of the cervical region due to edematization, as described above.

Therefore, it is inconsistent that hyaluronidase for decomposing hyaluronic acid important for maintaining the water holding property conversely facilitates ripening. Although the factual relations are vague, it is defined that hyaluronic acid is a biochemical marker for cervical ripening. Therefore, the fact found by the inventors that cervical ripening is induced by administering hyaluronic acid itself is a surprising matter. As a result of intensive research based on this finding, the inventors found that ripening reaction in the uterine cervix induced by hyaluronic acid is supported by not only a morphological ripening change of the uterine cervix but also other biochemical markers for cervical ripening, for example, the water content of the cervical region, an increase in collagenase activity, etc., and further found reaction for increasing the production of interleukin-8 in the uterine muscle, which is known as a biological factor for cervical ripening. This resulted in the completion of the invention relating to hyaluronic acid as a cervical ripening agent.

As described above, the methods for assisting the parturition process include the means for enhancing the delivery force, and the means for dilating the uterine cervix. The means for enhancing the delivery force include mechanical methods using a bougie, a colpeurynter and metreurynter, and means using medicines such as prostagandin E₂ and F_{2α}, oxytocin which is a posterior lobe hormone of hypophysis, an oxytocic hemostatic agent, an oxytocic agent, and the like. These methods are means for stimulating uterine contraction, and thus cause excessive contraction, excessive labor pains, shock, or the like in a pregnant woman. The physical operation of dilation by inserting a laminaria as a method of softening the uterine cervix has a danger of infection and the problem of damaging a mother or causing pains due to stimulation of the tunica mucosa uteri. The pharmacological method of ripening the uterine cervix using estriol requires intramuscular injection, and causes unnecessary pains in a mother before parturition. Similarly, prasterone sodium sulfate (Mylis) also requires intravenous injection at frequent intervals. At present, the use of a hormone formulation is restrained as much as possible from the viewpoint of adverse effects. In regard to biologically active substances belonging to cytokines, such as IL-8, IL-1_{β}, and the like, which have recently attracted attention, clinical evaluation has not been defined yet, and the production cost is thought to be high.

### Disclosure of Invention

The present invention relates to a cervical ripening agent containing as an active ingredient hyaluronic acid or a hyaluronic acid derivative, and a cervical dilator containing the ripening agent.

### Brief Description of the Drawings

Fig. 1 shows the dose-dependent effect of accelerating the production of hyalurounic acid in tissue culture of the uterine muscle of the human lower uterine segment when interleukin-8 was added.

Fig. 2 shows the dose-dependent accelerating effect on the activities of collagenase and gelatinase produced in a supernatant in tissue culture of the uterine muscle of the human lower uterine segment when hyaluronic acid was added.

Fig. 3 shows the accelerating effect on the content of interleukin-8 produced in a supernatant in tissue culture of the uterine muscle of the human lower uterine segment when hyaluronic acid was added.

Fig. 4 shows increases in the water content of the rabbit uterine cervix by intravaginal administration of hyaluronic acid.

### Best Mode for Carrying Out the Invention

Hyaluronic acid and/or hyaluronic acid derivatives used in the present invention may be obtained by separating and purifying natural hyaluronic acid and/or derivatives thereof by a general method, or formed by a known chemical synthetic method. In the present invention, hyaluronic acid and hyaluronic acid derivatives may be used for medical applications without other materials, or may contain an excipient, a binder, a lubricant, a colorant, a stabilizer, and the like, which are acceptable as medicines (DRUGS IN JAPAN, ETHICAL DRUGS, August, 1995, p. 990, Edited by JAPAN PHARMACEUTICAL INFORMATION CENTER).

Although the molecular weight of the hyaluronic acid used in the present invention is not limited, hyaluronic acid having a molecular weight of about 2000 to 500,000 is preferably used because of its strong maturating action on the uterine cervix. Hyaluronic acid derivatives are not limited as long as they have the same degree of action to ripen the uterine cervix as hyaluronic acid, but examples of such derivatives include hyaluronates and hyaluronic acid antibiotics. Examples of hyaluronates include esters of hyaluronic acid and aliphatic, aromatic or heterocyclic alcohols. In this case, the carboxyl groups of hyaluronic acid may be wholly or partially esterified.

The content of hyaluronic acid and/or a hyaluronic acid derivative of the ripening agent of the present invention depends upon the age of a pregnant woman as an administration object, states of the uterus, for example, the degree of rigidity of the soft parturient canal and health conditions of the body, the content is, for example, 1 ng to 10 g/kg, preferably 1 µg to 3000 mg/kg, more preferably 1 mg to 1000 mg/kg. The content is not limited to this range, and hyaluronic acid or a derivative thereof may be added in an amount necessary for exhibiting the intended pharmacological effect. Also the precise dose of hyaluronic acid and/or a hyaluronic acid derivative depends upon the age, body weight and sexuality of an object, and the type, properties and symptom stage of a disease. Therefore, the precise dose cannot previously be specified, and finally determined by a therapist.

The form of the cervical ripening agent of the present invention is not limited so long as the object of the present invention can be achieved, but the ripening agent can be formed into a liquid agent such as an intravaginal spray or the like, a semisolid agent such as an ointment, a cream, a gel, or the like; a solid agent such as a vaginal tablet, a vaginal capsule, a pessary, a vaginal suppository, or the like. Formulations which can be coated on a cervical dilator, such as a liquid agent and a semisolid agent, can also be administered by using the cervical dilator. The term "coating" means not only coating of an agent on the surface of the dilator but also impregnation of an agent using appropriate impregnating means provided on the dilator. In this way, by combining the cervical dilator with the cervical ripening agent of the present invention, dilation of the uterine cervix can more easily be achieved.

In order to prepare the above formulations, besides the above components, components used as raw materials for general medical formulations can be added to form target formulations. Examples of such components include animal and plant oils (soybean oil, beef tallow, synthetic glycerides, and the like), hydrocarbons (liquid paraffin, squalene, solid paraffin, and the like), ester oils (octyldodecyl myristate, isopropyl myristate, and the like), higher alcohols (cetostearyl alcohol, behenil alcohol, and the like), silicone resins, silicone oil, surfactants (polyoxyethylene fatty acid esters, sorbitan fatty acid esters, glycerin fatty acid esters, polyoxyethylene fatty acid esters, polyoxyethylene hardened castor oil, polyoxyethylene-polyoxypropylene block copolymers, and the like), water-soluble polymers (hydroxyethyl cellulose, polyacrylic acid, carboxyvinyl polymers, polyethylene glycol, polyvinylpyrrolidone, methyl cellulose, and the like), alcohols (ethyl alcohol, isopropyl alcohol, and the like), polyhydric alcohols (glycerin, propylene glycol, dipropylene glycol, sorbitol, and the like), saccharides (glucose, sucrose, and the like), inorganic powders (silicic anhydride, aluminum magnesium silicate, aluminum silicate, and the like), purified water and the like. In order to control pH, an inorganic acid (hydrochloric acid, phosphoric acid, or the like), an alkali metal salt of an inorganic acid (sodium phosphate or the like), an inorganic base (sodium hydroxide or the like), an organic acid (a lower fatty acid, citric acid, lactic acid, or the like), an alkali metal salt of an organic acid (sodium citrate, sodium lactate, or the like), an organic base (arginine, ethanolamine, or the like), or the like can be used. Also an antiseptic, an antioxidant, and the like can be used as occasion demands. Although actual additives are appropriately selected from the above additives or in combination of at least two additives according to the formulation of the therapeutic agent used for an object disease, of course, the additives are not limited to these additives.

As the cervical dilator of the present invention, besides plants such as Laminaria Japonica, and the like, dilators comprising other conventional synthetic resins (regardless of whether they are water absorbing resins or thermoplastic resins, whether they swells with water or not, and whether the substrates are spongy or fibrous), or polymer absorbers, and other dilators which can be used for the purpose may be used regardless of material and shape.

The above-described invention can also be carried out for animals other than humans. Namely, by applying the cervical ripening agent comprising hyaluronic acid as an active ingredient, and the cervical dilator coated or impregnated with the ripening agent, to animals, the ripening agent and the dilator can be used for curing, surgery and treatment of non-pregnant and pregnant animals. In other words, the present invention can be used for not only parturition of humans and animals other than humans, but also general therapeutic treatment and treatment before artificial fertilization treatment, which require dilation and ripening of the uterine cervix in non-pregnancy, and all operations comprising dilation and ripening of the uterine cervix in pregnancy. For example, the present invention can be applied to dilation and ripening of the uterine cervix before artificial abortion in the early stage of pregnancy and evacuation in the middle stage of pregnancy, and amelioration treatment for rigidity of the soft parturient canal in the latter stage of pregnancy. Furthermore, the present invention has the advantage of the action to dilate and ripen the uterine cervix without causing birth pains in treatment before labor induction.

Examples are described below for indicating the effect of the present invention. However, the examples are described only as examples, and should not be restrictively understood in any sense.

### [EXAMPLES]

### EXAMPLE 1

### Distributions of hyaluronic acid and CD44 (hyaluronic acid receptor) in the human uterine cervix:

### (Method)

(1) In sample preparation, tissues were collected from the uterine cervix of three patients by biopsy in cesarean section with written informed consents. The uterine cervix of all patients had a Bishop's score of 4 or less due to insufficient ripening. Also the cervical regions were collected from six patients who were subjected to uterectomy due to benign tumor and who showed a symptom that the focus did not reach the uterine cervix.
(2) In preparation of biotinylated hyaluronic acid-bound protein, a cartilage extract was dialyzed from a bovine nasal cartilage composed of hyaluronic acid-bound protein (abbreviated to HABP hereinafter) by the Knudson-Toole method (Knudson, W. and Toole, B. P., J. Cell Bio., 100, 1753, 1985), and then prepared to form a freeze-dried material. The freeze-dried material was treated with trypsin in a 0.1 M acetic acid-0.1 M Tris/hydrochloric acid buffer (pH 7.3) by the Tengblad's method (Tengblad, A., Biochem. Biophys. Acta, 578, 281, 1979), dialyzed with a Hepes buffer, freeze-dried and then dissolved in a Hepes buffer. After centrifugation, the supernatant was biotinylated by the Updike-Nicolson method (Updike, T. V. and Nicolson, G. L., Methods Enzymol., 121, 717, 1986) using 2 mM of Sulfo-NHS-biotin (Sigma St. Louis, Mo). The thus-obtained biotinylated protein was dialyzed with 0.4 M hydrochloric acid guanidine and 0.5 M sodium acetate, and then subjected to column chromatography using HA-sepharose. The HA-bound protein was eluted with 80 ml of 4 M hydrochloric acid guanidine.
(3) In immuno-staining of hyaluronic acid, a section of the diparaffinized cervix was incubated in methyl alcohol containing 2% of bovine serum albumin and 3% of hydrogen peroxide. After washing with PBS, the section was reacted with 100 µl of biotinylated hyaluronic acid-bound protein (biotinylated HABP) at 4 °C overnight. After three times of washing with PBS, the tissue section was incubated with strepto avidin-biotin-peroxidase diluted to 1 : 200 at room temperature for 30 minutes. The tissue section was then immersed in a solution containing 1.5 mg/ml of diaminobenzidine and 0.01% of hydrogen peroxide.
(4) Immuno-staining of CD44 (hyaluronic acid acceptor) was carried out by using CD44 monoclonal antibody (Serotec Ltd., Oxford, England) and avidinperoxidase complex in accordance with the Letarte's method (Letarte, M. Molecular Immunol., 23, 639, 1985).

### (Results)

Hyaluronic acid in the human cervical tissue was locally observed in the connective tissue, and not present in the smooth muscle region. Hyaluronic acid in the cervical canal tissue was uniformly present in the connective tissue. Hyaluronic acid was more present in the uterine cervix of the pregnant animals than the non-pregnant animals. Also, large amounts of hyaluronic acid were observed around the blood vessels, in the cervical glands of the cervix, and directly below the cervical epithelium. In investigation by immuno-staining, it was found that CD44 is uniformly distributed in the cervix of pregnant human and non-pregnant origin, and the amount of distribution in the cervix of the pregnant origin is larger than the cervix of the non-pregnant origin. Particularly, CD44 was richly present around the blood vessels and in the cervical glands. CD44 was detected as a strong CD 44 stained image in the basal layer of the cervical epithelium.

### EXAMPLE 2 Influence of interleukin-8 on synthesis of hyaluronic acid in the human uterine muscle

### (Method)

(1) In sample collection and tissue culture, an uterine muscular sample was collected from the lower uterine muscle of each of 15 patients by biopsy in cesarean section. The ages of the patients were 29 ± 2.4 years old, and the gestational ages in week were 38 ± 0.3 weeks. Each of the biopsy samples was lightly washed with warmed physiological saline to remove blood clots, and cut into small pieces. Tissue culture was carried out by using 100 mg of uterine muscle for 1 ml of medium (MEM) to which serum was not added. The amount of interleukin added was each of 50, 100, 150 and 200 ng/ml, and the hyaluronic acid content and hyaluronidase activity were measured after culture at 37 °C for 24 hours.
(2) In measurement of the hyaluronic acid content and hyaluronidase activity, the amount of hyaluronic acid was measured by a solid phase method using a 96-well plate. Namely, 100 µl of purified HABP (0.5 µg/ml) was input in each of the wells of the 96-well plate, and then allowed to stand at 4 °C for 16 hours to coat the surface of the plate. The plate was lightly washed, treated with a TBS buffer containing 2% of bovine serum albumin at room temperature for 1 hour and then blocked. 50 µl of each of the samples was added to each of the wells, followed by reaction at room temperature for 2 hours. After the plate was washed at least three times with a TBS buffer containing 0.05% Tween-20, 100 µl of TBS buffer containing 2% bovine serum albumin and 0.5 µg/ml of biotinylated HABP was added to each of the wells, followed by reaction at room temperature for 2 hours. Then abidine-peroxidase was added to each of the wells to color the sample. Quantitative determination was carried out by measuring absorbance at a wavelength of 450 nm by using Bio-Rad EIA Reader (Bio-Rad Model 2550, Bio-Rad Lab., Richmond, CA). All samples were measured in duplicate. The hyaluronidase activity was measured by substrate electrophores is using the culture supernatant of the uterine muscle and the homogenate of the uterine muscle as samples (Miura, R. O. et al., Analytical Biochemistry, 225, 333, 1995). As the substrate, chondroitin sulfate was used.
(3) In measurement of collagenase and gelatinase activities, each of 0, 0.5, 1 and 2 mg of hyaluronic acid (Sigma Co., St Louis, MO) derived from the human umbilical cord was dissolved in 1 ml of medium and added to a culture system of the human uterine muscle to carry out experiment. The collagenase and gelatinase activities in the supernatant were measured by using a commercial specific measurement kit (Collagenase type I activity measurement, and Gelatinase type IX activity measurement, Yagai Co., Cosmo Bio, Tokyo).

### (Results)

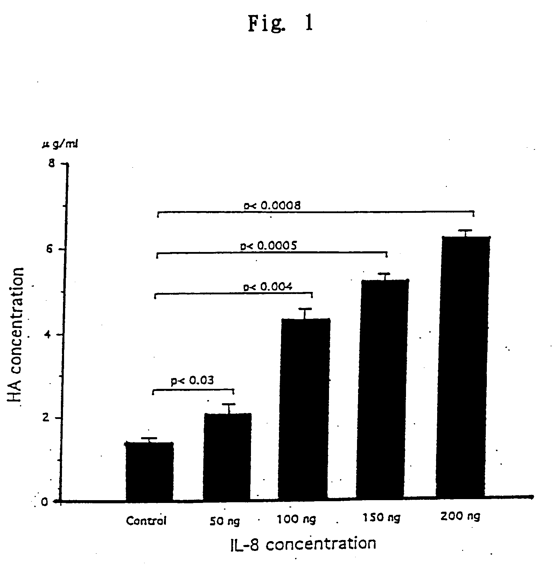

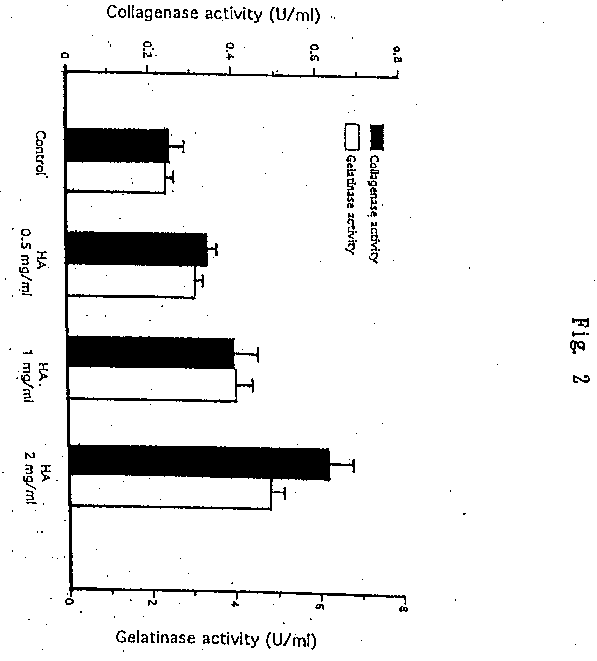

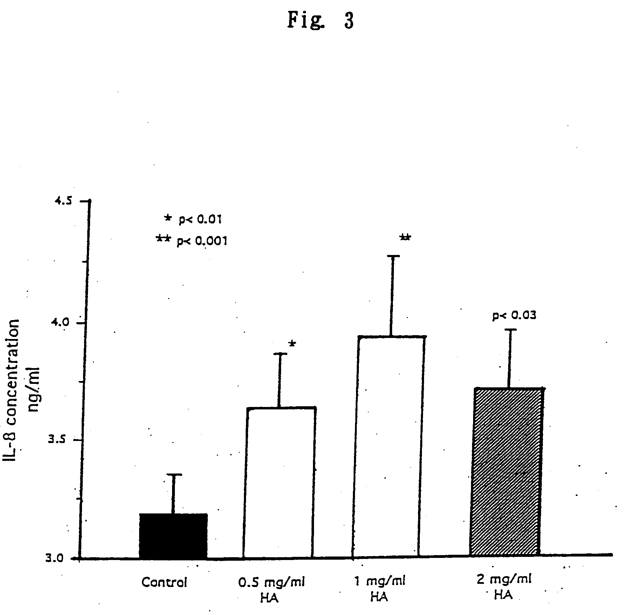

A relatively large amount of hyaluronic acid was synthesized even by culture of the lower tissue of a pregnant human uterine muscle. As a result of addition of interleutin-8 to this tissue culture system, the hyaluronic acid contents in the culture supernatant and the tissue homogenate were increased depending upon the dose of interleukin-8. Fig. 1 shows the result that the hyaluronic acid content in the supernatant is increased. The hyaluronidase activity was not greatly changed by stimulation with interleukin-8. As a result of addition of hyaluronic acid to the tissue culture system of the uterine muscle, increases in the collagenase activity depending upon the dose of hyaluronic acid were observed. Similarly, the gelatinase activity was increased due to reaction with hyaluronic acid depending upon the dose of hyaluronic acid (Fig. 2). Further, the addition of hyaluronic acid to the tissue culture system of the uterine muscle causes increases in the amount of interleukin-8 in the culture supernatant. The highest production of interleutin-8 was caused by adding 1 mg/ml of hyaluronic acid. As shown in Fig. 3, the production of interleukin-8 was accelerated even by adding 2 mg/ml of hyaluronic acid, but it was significantly lower (p < 0.05) than addition of 1 mg/ml of hyaluronic acid.

### EXAMPLE 3

### Action of hyaluronic acid on the rabbit uterine cervix:

### (Method)

In experiment, eight New Zealand White (NZW) rabbits having a body weight of 3 to 3.5 kg were used as each of pregnant and non-pregnant groups. The pregnant rabbits were primigravidae and on the 23rd day of pregnancy. Hyaluronic acid (Sigma St. Louis MO, USA) derived from human umbilical cords was mixed with a viscous base (adepos solidus, Mitusba Co. Tokyo) and administered into the vagina of each rabbit once a day so that the dosage was 20 mg/head/day. At the third day, the cervix was isolated from each of the rabbits, and the hardness and dilation of the cervix were measured by using a Hegar's dilator. The water content of the cervix was measured at different four positions in the cervix by using an infrared moisture meter IM-3SCV (Fuji Technica Co., Osaka) according to the method disclosed in the document (Sumitomo, K. and Terao, T., 1993, Jpn. Soc. ME&BE., 32, 337), and an average water content was determined. Further, the uterine cervix was fixed by a 10% neutral buffered formalin fixative, embedded in paraffin, and then cut at a position (the center of the uterine cervix) of about 0.3 mm from the external as to form a section. For the section, a change in the cervical structure was observed by hematoxylin-eosin staining, or the state of collagen was observed by picro Sirius red staining (Junqueira, L. C. V. et al., 1980, Am. J. obstet. Gynecol., 138, 273).

### (Results)

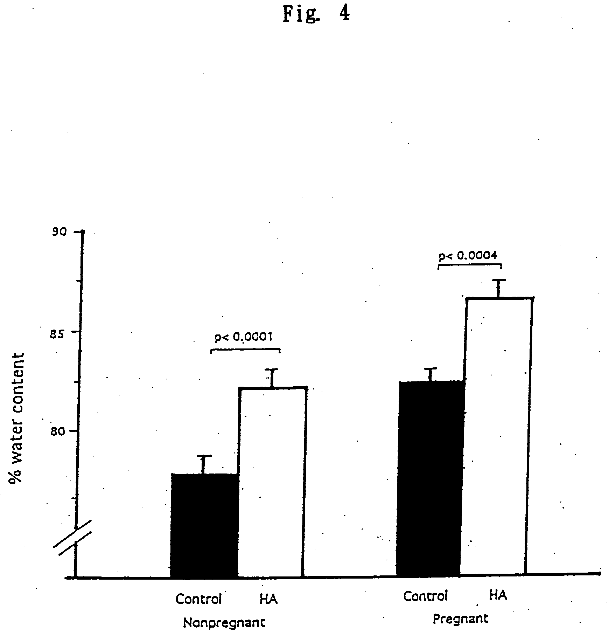

In both the pregnant and non-pregnant rabbits, the uterine cervix were ripened by the administration of hyaluronic acid. Namely, the color tone became violet, and the macroscopically softened cervix could be easily dilated by the Hegar's dilator. The water content was also increased in the group to which hyaluronic acid was administered (Fig. 4). In Fig. 4, "Control" indicates placebo, and HA indicates hyaluronic acid. In hematoxylin -eosin staining, the cervix of the group to which hyaluronic acid was administered showed an edema state, and the substrates were increased to divide the collagen fibers. In both the pregnant and non-pregnant rabbits, many extended blood vessels filled with the erythrocytes were observed in the connective tissue, and mild neutrophil migration was observed in the connective tissue. On the other hand, in the non-pregnant rabbits to which placebo was administered, the collagen fibers stained with picro Sirius red had firmly maintain the structure, and densely distributed as fascicular fibers. In the pregnant rabbits to which placebo was administered, the collagen fascicular fibers were smoothly distributed, and dissociated from each other due to an increase in the fiber spacing. In the pregnant and non-pregnant rabbits to which hyaluronic acid was administered, the collagen fascicular fibers were dissociated, and changed to a network structure formed by fine fiber fragments. The collagen fibers which formed the network had no directivity. Also, the spacing of the collagen fibers was significantly increased by edematization.

### Industrial Applicability

The present invention discloses the cervical ripening action of hyaluronic acid and/or hyaluronic acid derivatives. Proof of the same action by not only pregnant animals but also non-pregnant animals exhibits effectiveness of hyaluronic acid and/or hyaluronic acid derivatives as medicines and application means for not only parturition and abortion treatment but also treatment related to artificial fertilization was exhibited.

## Claims

1. A cervical ripening agent comprising as an active ingredient at least one selected from the group consisting of hyaluronic acid and hyaluronic acid derivatives.

2. A cervical ripening agent according to Claim 1, wherein hyaluronic acid and hyaluronic acid derivatives are derived from natural substances or chemical synthetic substances.

3. A cervical ripening agent according to Claim 2, the molecular weight of hyaluronic acid is 2000 to 500000.

4. A cervical ripening method comprising administering a cervical ripening agent comprising as an active ingredient at least one selected from the group consisting of hyaluronic acid and hyaluronic acid derivatives.

5. A cervical ripening method according to Claim 4, wherein the molecular weight of hyaluronic acid is 2000 to 500000.

6. A cervical ripening method according to Claim 4 or 5, wherein the dose is 1 ng to 1000 mg/kg.

7. A cervical ripening method according to Claim 4 or 5, comprising administering through a cervical dilator.

8. A cervical dilator comprising hyaluronic acid and/or a hyaluronic acid derivative coated thereon or contained therein.

9. A cervical dilator according to Claim 8, wherein the molecular weight of hyaluronic acid is 2000 to 500000.

10. A treatment method using hyaluronic acid and/or a hyaluronic acid derivative for ripening the uterine cervix in parturition, abortion or artificial fertilization of a human.

11. A treatment method according to Claim 10, wherein the molecular weight of hyaluronic acid is 2000 to 500000.

12. A treatment method for an animal other than a human using hyaluronic acid and/or a hyaluronic acid derivative for ripening the uterine cervix in parturition, abortion or artificial fertilization of an animal other than a human.

13. A treatment method according to Claim 12, wherein the molecular weight of hyaluronic acid is 2000 to 500000.
